# EUROPEAN PATENT SPECIFICATION

(11) **EP 2 443 924 B1**
(45) Date of publication and mention of the grant of the patent: **22.06.2016**
(21) Application number: 11182662.4
(22) Date of filing: 26.09.2011
(51) Int. Cl.: A01N 25/34, A01N 43/40, A61K 8/44, A61K 8/02, A61K 8/49, A61Q 17/00, D06M 16/00, D06M 101/20, D06M 101/32

(54) **Textile sheetlike structure impregnated with an antimicrobial active ingredient solution for use on animate surfaces**
Mit antimikrobieller Wirkstofflösung imprägnierte bahnförmige textile Struktur zur Verwendung auf belebten Oberflächen
Structure textile de type feuille imprégnée par une solution d'ingrédient actif antimicrobien à utiliser sur des surfaces animées

(30) Priority: 22.10.2010 DE 102010049114
(43) Date of publication of application: 25.04.2012
(73) Proprietor: L'Air Liquide Société Anonyme pour l'Etude et l'Exploitation des Procédés Georges Claude, 75007 Paris (FR); Schülke & Mayr GmbH, 22851 Norderstedt (DE)
(72) Inventor: Dettmann, Andreas, 22157 Hamburg (DE); Spuida, Thomas, 22419 Hamburg (DE); Behrends, Sabine, 25482 Appen (DE); Wolff, Michael, 20251 Hamburg (DE)
(74) Representative: Conan, Philippe Claude

(56) References cited:
- EP-A1- 2 241 336
- WO-A1-97/16066
- US-A- 4 100 324
- US-A- 5 141 803
- Rhodia Corp.: "MIRANOL C2M CONC NP - Product data sheet N000861 - Disodium cocoamphodiacetate", , 1 March 2008 (2008-03-01), pages 1-2, XP55018545, Retrieved from the Internet: URL:http://www.innovadex.com/documents/645 57.pdf?bs=1529&b=38508&st=20 [retrieved on 2012-02-07]
- N O HÜBNER ET AL: "Octenidine Dihydrochloride, a Modern Antiseptic for Skin, Mucous Membranes and Wounds", SKIN PHARMACOLOGY AND PHYSIOLOGY, vol. 23, no. 5, 18 May 2010 (2010-05-18), pages 244-258, XP55018202, Basel ISSN: 1660-5527, DOI: 10.1159/000314699

## Description

The invention relates to a textile sheet-like structure impregnated with an antimicrobial active ingredient solution for use on animate surfaces, for example on human skin. The active ingredient solution comprises N,N'-(1,10-decanediyldi1[4H]-pyridinyl-4-ylidene)bis(1-octanamine) dihydrochloride (octenidine dihydrochloride, hereinbelow octenidine). Furthermore, the invention relates to the use of additives in such an active ingredient solution for reducing the adsorption of the bispyridiniumalkane on synthetic support materials. Finally, a kit of synthetic support material and antimicrobial active ingredient solution for producing such sheet-like materials is also provided.

Disinfectant active ingredient solutions for hygienic hand disinfection according to EN 1500 and for disinfecting hand washing according to EN 1499 are known. Thus, for example, Schülke & Mayr GmbH, Norderstedt (Federal Republic of Germany) sells an aqueous composition for hand washing which comprises 0.3% by weight of the antimicrobially effective bis-pyridiniumalkane *octenidine dihydrochloride* (hereinbelow octeninidine), and also inter alia glycerol and amine oxides as auxiliaries.

Moreover, wet wipes are described in the prior art for use on the human skin. For example, there are wet wipes for use with small children, for intimate care and for the cleaning and total-bodycare of bed-ridden persons and those requiring full care. These wet wipes usually comprise substances which clean and care for the skin. For example, the wet wipes of the Menalind^{®} professional project series from Paul Hartmann AG, Heidenheim (Federal Republic of Germany) comprise an alcohol-free care liquid containing creatine and moisture-donating panthenol. For odour neutralization, a deodorizing composition is also present (cf. EP 401 140 B1). The deodorizing composition comprises a combination of two aldehydes. However, aldehydes are of concern as active ingredients for use on the skin from an allergological point of view.

Moreover, solutions of antimicrobial active ingredients applied to support materials for the disinfection of inanimate surfaces are known. However, support materials impregnated with active ingredient solutions may result in the adsorption of active ingredients, particularly if the active ingredients are quaternary ammonium compounds. This problem of adsorption of active ingredients on carrier materials also arises with the antimicrobially effective bispyridiniumalkanes.

EP 1 661 586 A1, which deals with the disinfection of hard surfaces, proposes, for the purpose of preventing active ingredient adsorption, the use of a support material made of plastic fibres, for example polyethylene terephthalate (PET). However, it has been found that certain active ingredients, which also include bispyridiniumalkanes, are adsorbed even when using PET. The problem of active ingredient adsorption on fibres of the support material is thus not solved by using PET. In particular, EP 1 661 586 A1 reveals no teaching as to how a wet wipe for use on animate surfaces such as human skin can be produced using bispyridiniumalkanes as active ingredients.

WO 2004/000373 A1, which likewise deals with the disinfection of hard surfaces, accordingly proposes introducing specific additives which attach to the fibres of the support materials in order to prevent the adsorption of active ingredients. The additives proposed are specifically quaternary ammonium compounds, polydialkyldiallylammonium salts with acrylamide and/or acrylic acid and/or vinyl acetate and derivatives thereof. The obligatory use of these additives thus limits the options of formulating an active ingredient solution with the lowest possible amount of quaternary ammonium compound, and is therefore not contemplated for applications on the skin.

EP 2 241 336 A1 discloses a disinfecting substrate comprising a support made of synthetic material (a) and a preparation (b) containing one or more antimicrobial active substances selected from the group of quaternary ammonium compounds, guanidine, alkylamines and/or their derivatives, aldehydes and/or phenol).

Although, during the production of wet wipes it would in principle be possible, for the purpose of overcoming the disadvantage of the adsorption of active ingredient on the support material, to work with an excess of the active ingredient in the solution, this procedure is problematic if the support materials impregnated with active ingredient solutions are then to be used on the skin. The increase in the concentration of the active ingredient is limited in this case on account of possible incompatibilities.

The object of the present invention was therefore to provide textile sheet-like materials for the cleaning and disinfection of animate surfaces (in particular human skin). The sheet-like materials should be impregnated with a solution based on octenidine, as active ingredient, but the described adsorption of active ingredient on the carrier material should not result. The impregnated sheet-like structures should also be able to be formulated on the basis of a large number of support materials. Moreover, it should be possible for active ingredient solutions to be formulated in diverse ways. Ultimately, the obligatory presence of additives which only serve to prevent adsorption, but the presence of which offers no further advantages (as is the case for the additives in WO 2004/000373 A1) should not be necessary.

It has now surprisingly been found that these and further objects are achieved through the use of certain additives in the solutions of octenidine.

Accordingly, the invention relates firstly to a textile sheet-like structure impregnated with an antimicrobial active ingredient solution according to Claim 1.

The invention is based inter alia on the fact that it has surprisingly been found that the specified additives prevent the adsorption of octenidine on synthetic support materials. Here, the additives are multifunctional, i.e. they impart additional advantageous properties to the active ingredient solution (besides the effect preventing adsorption).

The term "synthetic support material" refers to a manmade fibre material which, besides the polymer, can comprise typical plastics additives. The term thus includes manmade fibre material based on natural polymer (such as polylactide fibres) and manmade fibre material based on synthetic polymer. Synthetic polymers are polyethylene terephthalate (PET), and polypropylene (PP), and mixtures of both polymers. Besides the synthetic support material based on synthetic polymer, the sheet-like structure can also contain support material based on natural polymer. In one preferred embodiment, however, in the sheet-like structure according to the invention - besides the synthetic support material based on synthetic polymer - the presence of synthetic support material based on natural polymer is excluded.

Likewise preferably excluded is the presence of natural fibres (such as plant fibres, fibres of animal origin and mineral fibres).

The support material is preferably used as nonwoven. All nonwovens are suitable. Preference is given to nonwovens consolidated thermally or by water-jet methods. Particular preference is given to nonwovens consolidated by water-jet methods (spunlace), which are characterized by a particularly high absorbency.

Typical embodiments of the support material are gloves and wipes.

The typical total area of the glove is 100-2000 cm², preferably 300-1000 cm², in particular 500-850 cm², for example 640-770 cm², such as for example 690 cm².

If the carrier material is in the form of a glove, then the glove may be, for example, a mitten, three-finger glove or five-finger glove. In a preferred embodiment, however, the glove is produced as a simple mitten from the following amount of support material:

| | Width (cm) | Length (cm) |
|---|---|---|
| Preferably | 11-21 | 30-56 |
| More preferably | 13.5-18.5 | 36.5-49.5 |
| In particular | 14.5-17.5 | 39-47 |
| For example | about 16 | about 43 |

To produce such a simple mitten, the total area is typically placed one on top of the other and sewn or (thermally or adhesively) glued at the two sides in order to produce the glove which, as an outside dimension, has approximately the stated width x half of the stated length.

If the support material is in the form of a wipe, then the typical area is 50-2000 cm², preferably 100-1000 cm², more preferably 300-900 cm², in particular 400-800 cm², for example about 600 cm². Typical sizes of the wipe are in the following ranges:

| | Width (cm) | Length (cm) |
|---|---|---|
| Preferably | 14-26 | 21-39 |
| More preferably | 17-23 | 25.5-34.5 |
| In particular | 18-22 | 27-33 |
| For example | about 20 | about 30 |

In one particularly preferred embodiment, besides a support material made of (i) polyester, in particular PET, no further support material is present. If PET nonwoven is used as support material, then typical areal weights are > 15 g/m². Preferably, the areal weight when using PET as support material is in a range from 30-130 g/m², more preferably 50-110 g/m², in particular 60-100 g/m², such as, for example, about 80 g/m². A typical embodiment when using PET as support material is a glove with the total area stated above.

In a further particularly preferred embodiment, besides a support material made of (ii) polyolefin, in particular PP, no further support material is present. If polyolefin nonwoven, in particular PP, is used as support material, then typical areal weights are likewise > 15 g/m². Preferably, the areal weight when using PP as support material is in a range from 28-125 g/m², more preferably 45-105 g/m², in particular 55-95 g/m², such as, for example, about 75 g/m². A typical embodiment when using polypropylene as support material is a wipe having the area stated above.

The textile sheet-like structures according to the invention moreover comprise b) an antimicrobial active ingredient solution. This active ingredient is N,N'-(1,10-decanediyldi-1[4H]-pyridinyl-4-ylidene)bis(1-octanamine) dihydrochloride (octenidine dihydrochloride, hereinbelow octenidine).

A typical amount of component b1) is 0.02 to 5% by weight, preferably 0.03 to 3% by weight, in particular 0.04 to 0.5% by weight, for example 0.06 to 0.2% by weight, such as, for example, 0.08% by weight.

Besides the active ingredient component b1), the active ingredient solution used according to the invention comprises b2) one or more additives selected from b2a) nonionic and b2b) amphoteric surfactants. Typically, the total amount of component b2) is 0.03 to 10% by weight, preferably 0.06 to 5% by weight, in particular 0.1 to 2% by weight.

According to the invention, as b2a) nonionic surfactants are (iii) alkyl polyglucosides and (iv) amine oxides, in particular (iv) amine oxides.
(iii) alkyl glycoside is a C₈- to C₁₆-alkyl polyglucose of a fatty alcohol, preference being given to a lauryl polyglucose, a decyl polyglucose or a mixture thereof. The carbon chain length for the cocoyl polyglucose is 8 to 16 atoms, for the laurylpolyglucose is 12 to 16 carbon atoms and for the decyl polyglucose is likewise 8 to 16 carbon atoms.

A typical amount of alkyl glycoside is 0.03 to 10% by weight, preferably 0.06 to 5% by weight, in particular 0.1 to 2% by weight.

According to the invention, the amine oxide has the general formula

R¹R²R³N-O,

in which R¹ is methyl, ethyl or 2-hydroxyethyl, R² is methyl, ethyl or 2-hydroxyethyl, R¹ and R² together may be morpholine, R³ is alkyl having 8 to 18 carbon atoms or R⁴CONH(CH₂)ₙ, where R⁴ is alkyl having 8 to 18 carbon atoms and n is in the range from 1 to 10, preferably 1 to 5, more preferably 2 to 4, and in particular 3.

Examples of amine oxides are cocamidopropylamine oxide, N-cocomorpholine oxide, decadimethylamine oxide, dimethylcetylamine oxide, dimethylcocamine oxide, dimethyl-hydr. tallow amine oxide, dimethyllaurylamine oxide, dimethylmyristylamine oxide, (2-hydroxyethyl)cocamine oxide and oleamine oxide. See also "International Cosmetic Ingredient Dictionary and Handbook", 10th edition 2004, volume 3, pages 2268-2275 (Surfactants-Cleansing Agents).

In one preferred embodiment, the amine oxide is cocamidopropylamine oxide, i.e. R⁴CO is the acyl radical derived from the fatty acids of coconut oil, n = 3, and R¹ and R² are methyl. This product is sold as Rewominox B 204 by Goldschmidt, Federal Republic of Germany.

A typical amount of amine oxide is 0.03 to 10% by weight, preferably 0.06 to 5% by weight, in particular 0.1 to 2% by weight.

The amphoteric surfactant b2b) is a betaine which is cocamidopropylbetaine. A typical amount of betaine is 0.03 to 10% by weight, preferably 0.06 to 5% by weight, in particular 0.1 to 2% by weight.

Moreover, the active ingredient solution used according to the invention comprises b3) water.

Finally, further auxiliaries may be present in the active ingredient solution, for example
(i) polyol, such as glycerol, in an amount of from 0.5 to 10% by weight, preferably 1 to 5% by weight, more preferably 1.5 to 3% by weight, such as 2 to 2.8% by weight, and
(ii) glycerol monoalkyl ethers, such as 1-(2-ethylhexyl) glycerol ether in an amount of from 0.01 to 5% by weight, preferably 0.02 to 2% by weight, in particular 0.03 to 0.5% by weight, such as 0.04 to 0.06% by weight.

The effect described above, namely that through the addition according to the invention of the specified additives, the adsorption of octenidine to synthetic support materials is prevented, is present at the typical pH values of active ingredient solutions which are contemplated according to the invention. Preferred pH values of the active ingredient solutions are in the range from 3 to 9, more preferably 4 to 8, such as 4.5 to 7, for example about 5.5. The desired pH can be adjusted, for example, using sodium lactate, citric acid or NaOH.

The antimicrobial active ingredient solution is preferably a solution which comprises
b1) octenidine in an amount of from 0.01 to 4% by weight, preferably 0.02 to 2% by weight, in particular 0.04 to 1.5% by weight, such as 0.06 to 0.2% by weight, for example about 0.08% by weight,
b2) amine oxide, preferably coconut fatty acid amidopropyldimethylamine oxide, as additive, preferably in an amount of from 0.03 to 10% by weight, more preferably 0.06 to 5% by weight, in particular 0.1 to 2.0% by weight, such as about 0.2% by weight,
b3) water as solvent and
b4) allantoin, glycerol, 1-(2-ethylhexyl) glycerol ether and sodium lactate as auxiliaries,
where a preferred active ingredient solution consists of components b1) to b4).

The antimicrobial active ingredient solution is likewise preferably a solution which comprises
b1) octenidine in an amount of from 0.01 to 4% by weight, preferably 0.02 to 2% by weight, in particular 0.04 to 1.5% by weight, such as 0.06 to 0.2% by weight, for example 0.08% by weight,
b2) cocamidopropylbetaine, as additive, preferably in an amount of from 0.03 to 10% by weight, more preferably 0.06 to 5% by weight, in particular 0.1 to 2.0% by weight, such as about 0.2% by weight,
b3) water as solvent and
b4) allantoin, glycerol, 1-(2-ethylhexyl) glycerol ether and sodium lactate as auxiliaries,
where a preferred active ingredient solution consists of components b1) to b4). Finally, it is preferred if the support material a) is impregnated with 1 to 12 times the weight of the active ingredient solution b), preferably 1.5 to 8 times, such as 2 to 5 times (weight/weight).

The impregnated sheet-like structures according to the invention are used in the customary manner on animate surfaces, in particular human skin. Because the adsorption of octenidine on the support material is prevented through the use of the specified additives, the use of the sheet-like structures leads neither to diminished effectiveness of the active ingredient solution, nor to incompatibilities (as could arise, for example, when using the additives according to WO 2004/000373 A1).

Secondly, the invention relates to the use of additives selected from nonionic and amphoteric surfactants in an antimicrobial active ingredient solution which, besides the specified additives, also comprises octenidine and water, in the production of textile sheet-like structures impregnated with the active ingredient solution for reducing the adsorption of octenidine on the synthetic support material used.

Thirdly, the invention relates to a kit for producing textile sheet-like structures impregnated with an antimicrobial active ingredient solution according to Claim 5.

Moreover, the disclosure relates to the aforementioned active ingredient solution for use for the disinfection of animate surfaces, in particular human skin, where the active ingredient solution as given above is present in the form of a textile sheet-like structure impregnated therewith. Moreover, the disclosure relates to octenidine for use for the disinfection of animate surfaces, in particular human skin, where octenidine is present in the form of an antimicrobial active ingredient solution in a textile sheet-like structure impregnated therewith.

The advantages of the present invention arise in particular from the following examples. In the examples and in the description above, all of the percentages refer to the total weight of the active ingredient solution.

### Example 1

Test design: 9 PET gloves (80 g/m²) and 12 PP wipes (75 g/m²) were impregnated with 3.25 times the weight of impregnation solution, stored for 24 hours in stacking boxes and then squeezed using a stainless steel potato press (WMF). During the squeezing of the wipes/gloves, in each case a squeezing-out rate of ca. 30-50% of the impregnation amount originally used was advised.

| | **A (comp.)** | **B (comp.)** | **C** | **D** | **E** | **F** | **G** |
|---|---|---|---|---|---|---|---|
| Purified water | 96.875 | 96.875 | 94.535 | 91.535 | 94.285 | 91.875 | 91.875 |
| Octenidine dihydrochloride | 0.050 | 0.050 | 0.050 | 0.050 | 0.050 | 0.050 | 0.050 |
| Glycerol 85% | 2.850 | 2.850 | 2.850 | 2.850 | 2.850 | 2.850 | 2.850 |
| Rewominox B 204 | | | 2.000 | 5.000 | | 5.000 | 5.000 |
| Cocamidopropylbetaine 30% | | | | | 2.500 | | |
| Oxadermol | 0.125 | 0.125 | 0.125 | 0.125 | 0.125 | 0.125 | 0.125 |
| Allantoin | 0.10 | 0.10 | 0.10 | 0.10 | 0.10 | 0.10 | 0.10 |
| Sodium lactate solution 50% | | | 0.34 | 0.34 | 0.09 | | |
| Citric acid | x | | | | | x | |
| NaOH 10% | | x | | | | | x |
| pH | 4.0 | 8.0 | 5.5 | 5.5 | 5.5 | 4.0 | 8.0 |
| | Octenidine [%] | Octenidine [%] | Octenidine [%] | Octenidine [%] | Octenidine [%] | Octenidine [%] | Octenidine [%] |
| Blank value | 0.052 | 0.052 | 0.050 | 0.054 | 0.052 | 0.053 | 0.052 |
| PET gloves | 0.040 | 0.043 | 0.052 | 0.054 | 0.052 | 0.054 | 0.051 |
| PP wipes | 0.048 | 0.046 | 0.052 | 0.054 | 0.052 | 0.054 | 0.051 |

### Example 2

Test design: In each case 10 PET gloves (80 g/m²), PP wipes (75 g/m²) and PET wipes (45 g/m²) were impregnated with 3.25 times the weight of impregnation solution, stored for 24 hours in stacking boxes and then squeezed using a stainless steel potato press. During the squeezing of the wipes/gloves, in each case a squeezing-out rate of 30-40% of the impregnation amount originally used was advised.

| | H (comp.) | I | J | K |
|---|---|---|---|---|
| | w (%) | w (%) | w (%) | w (%) |
| Purified water | 96.535 | 95.875 | 96.675 | 96.675 |
| Octenidine dihydrochloride | 0.050 | 0.050 | 0.050 | 0.050 |
| Glycerol 85% | 2.850 | 2.850 | 2.850 | 2.850 |
| Rewominox B 204 | | | 0.200 | 0.200 |
| Oxadermol | 0.125 | 0.125 | 0.125 | 0.125 |
| Allantoin | 0.100 | 0.100 | 0.100 | 0.100 |
| Sodium lactate solution 50% | 0.340 | | | |
| C12-C16 fatty alcohol polyglycoside (ca. 50%) | | 1.000 | | |
| 10% strength NaOH solution | | | | x |
| 10% strength citric acid solution | | x | x | |
| Total: | 100.000 | 100.000 | 100.000 | 100.000 |
| pH: | 5.5 | 5.5 | 4.0 | 8.2 |
| Content of octenidine in % blank value | 0.051 | 0.050 | 0.052 | 0.052 |
| Content of octenidine in %/squeezed-out solution PET glove format | 0.040 | 0.049 | 0.048 | 0.048 |
| Content of octenidine in %/squeezed-out solution PP wipe format | 0.046 | 0.048 | 0.048 | 0.047 |
| Content of octenidine in %/squeezed-out solution PET wipe format | 0.044 | 0.048 | 0.049 | 0.047 |

### Example 3

Test design: In each case 10 PET gloves (80 g/m²), PP wipes (75 g/m²) and PET wipes (45 g/m²) were impregnated with 3.25 times the weight of impregnation solution, stored for 24 hours in stacking boxes and then squeezed using the stainless steel potato press. During the squeezing of the wipes/gloves, in each case a squeezing-out rate of 30-40% of the impregnation amount originally used was advised.

| | **L (comp.)** | **M** | **N** | **O** | **P** | **Q** |
|---|---|---|---|---|---|---|
| | w (%) | w (%) | w (%) | w (%) | w (%) | w (%) |
| Purified water | 96.535 | 94.535 | 96.335 | 96.125 | 90.125 | 96.335 |
| Octenidine dihydrochloride | 0.050 | 0.050 | 0.050 | 0.050 | 0.050 | 0.050 |
| Glycerol 85% | 2.850 | 2.850 | 2.850 | 2.850 | 2.850 | 2.850 |
| Rewominox B 204 | | 2.000 | 0.200 | | | |
| Cocamidopropylbetaine 30% | | | | 0.660 | 6.660 | |
| Oxadermol | 0.125 | 0.125 | 0.125 | 0.125 | 0.125 | 0.125 |
| Allantoin | 0.10 | 0.10 | 0.10 | 0.10 | 0.10 | 0.100 |
| Sodium lactate solution 50% | 0.34 | 0.34 | 0.34 | 0.09 | 0.09 | 0.340 |
| C12-C16 fatty alcohol polyglycoside (ca. 50%) | | | | | | 0.20 |
| 10% strength citric acid | | | | | | x |
| Total: | 100.000 | 100.000 | 100.000 | 100.000 | 100.000 | 100.000 |
| pH: | 5.6 | 5.6 | 5.5 | 5.5 | 5.5 | 5.4 |
| Content of octenidine in %, blank value | 0.054 | 0.055 | 0.053 | 0.052 | 0.051 | 0.056 |
| Content of octenidine in %/squeezed-out solution PET glove format | 0.040 | 0.052 | 0.048 | 0.047 | 0.050 | 0.051 |
| Content of octenidine in %/squeezed-out solution PP wipe format | 0.048 | 0.052 | 0.049 | 0.049 | 0.050 | 0.051 |
| Content of octenidine in %/squeezed-out solution PET wipe format | 0.045 | 0.052 | 0.049 | 0.049 | 0.051 | 0.051 |

## Claims

1. Textile sheet-like structure impregnated with an antimicrobial active ingredient solution which comprises
a) a synthetic support material made of polypropylene, of polyethylene or of a mixture of both polymers, and
b) an antimicrobial active ingredient solution which comprises:
b1) 0.02% to 5% by weight octenidine,
b2) 0.03% to 10% by weight of an additive selected from an amine oxide of the general formula
R¹R²R³N-O,
in which R¹ is methyl, ethyl or 2-hydroxyethyl, R² is methyl, ethyl or 2-hydroxyethyl, R¹ and R² together may be morpholine, R³ is alkyl having 8 to 18 carbon atoms or R⁴CONH(CH₂)ₙ, where R⁴ is alkyl having 8 to 18 carbon atoms and n is in the range from 1 to 10, a C₈- to C₁₆-alkyl-polyglucose of a fatty alcohol or cocamidopropylbetaine; and
b3) water.

2. Sheet-like structure according to Claim 1, **characterized in that** the C₈-C₁₆-alkylpolyglucose is a laurylpolyglucose, a decylpolyglucose or a mixture thereof.

3. Sheet-like structure according to Claim 1, **characterized in that** the amine oxide is cocamidopropylamine oxide.

4. Sheet-like structure according to one of Claims 1 to 3, **characterized in that** the antimicrobial active ingredient solution futher comprises
b4) glycerol in an amount from 0.5% to 10% by weight and 1-(2-ethylhexyl) glycerol ether in an amount from 0.01% to 5% by weight.

5. Kit for producing textile sheet-like structures impregnated with an antimicrobial active ingredient solution which comprises
a) a synthetic support material made of polypropylene, of polyethylene or of a mixture of both polymers, and
b) an antimicrobial active ingredient solution which comprises:
b1) 0.02% to 5% by weight octenidine,
b2) 0.03% to 10% by weight of an additive selected from an amine oxide of the general formula
R¹R²R³N-O,
in which R¹ is methyl, ethyl or 2-hydroxyethyl, R² is methyl, ethyl or 2-hydroxyethyl, R¹ and R² together may be morpholine, R³ is alkyl having 8 to 18 carbon atoms or R⁴CONH(CH₂)ₙ, where R⁴ is alkyl having 8 to 18 carbon atoms and n is in the range from 1 to 10, a C₈- to C₁₆-alkyl polyglucose of a fatty alcohol or cocamidopropylbetaine; and
b3) water.

6. Kit according to Claim 5, **characterized in that** the C₈-C₁₆-alkylpolyglucose is a laurylpolyglucose, a decylpolyglucose or a mixture thereof.

7. Kit according to Claim 5, **characterized in that** the amine oxide is cocamidopropylamine oxide.

8. Kit according to one of Claims 5 to 7, **characterized in that** the antimicrobial active ingredient solution further comprises
b4) glycerol in an amount from 0.5% to 10% by weight and 1-(2-ethyl hexyl) glycerol ether in an amount from 0.01% to 5% by weight.

## Patentansprüche

1. Textile bahnähnliche Struktur, die mit einer antimikrobiellen Wirkstofflösung imprägniert ist, die Folgendes umfasst
a) ein synthetisches Trägermaterial, das aus Polypropylen, aus Polyethylen oder einer Mischung von beiden Polymeren hergestellt ist, und
b) eine antimikrobielle Wirkstofflösung, die Folgendes umfasst:
b1) 0,02 Gew.-% bis 5 Gew.-% Octenidin,
b2) 0,03 Gew.-% bis 10 Gew.-% eines Zusatzstoffs, der ausgewählt ist aus einem Aminoxid der folgenden allgemeinen Formel
R¹R²R³N-O,
wobei R¹ Methyl, Ethyl oder 2-Hydroxyethyl ist, R² Methyl, Ethyl oder 2-Hydroxyethyl ist, R¹ und R² zusammen Morpholin sein können, R³ Alkyl mit 8 bis 18 Kohlenstoffatomen ist, oder R⁴CONH(CH₂)ₙ, wobei R⁴ Alkyl mit 8 bis 18 Kohlenstoffatomen ist und n im Bereich von 1 bis 10 liegt, einer C₈- bis C₁₆-Alkylpolyglucose eines Fettalkohols oder Cocamidopropylbetain; und
b3) Wasser.

2. Bahnähnliche Struktur nach Anspruch 1, **dadurch gekennzeichnet, dass** die C₈-C₁₆-Alkylpolyglucose eine Laurylpolyglucose, eine Decylpolyglucose oder eine Mischung davon ist.

3. Bahnähnliche Struktur nach Anspruch 1, **dadurch gekennzeichnet, dass** das Aminoxid Cocamidopropylaminoxid ist.

4. Bahnähnliche Struktur nach einem der Ansprüche 1 bis 3, **dadurch gekennzeichnet, dass** die antimikrobielle Wirkstofflösung ferner Folgendes umfasst b4) Glycerin in einer Menge von 0,5 Gew.-% bis 10 Gew.-% und 1-(2-Ethylhexyl)glycerinether in einer Menge von 0,01 Gew.-% bis 5 Gew.-%.

5. Kit zum Herstellen textiler bahnähnlicher Strukturen, die mit einer antimikrobiellen Wirkstofflösung imprägniert sind, die Folgendes umfasst
a) ein synthetisches Trägermaterial, das aus Polypropylen, aus Polyethylen oder einer Mischung von beiden Polymeren hergestellt ist, und
b) eine antimikrobielle Wirkstofflösung, die Folgendes umfasst:
b1) 0,02 Gew.-% bis 5 Gew.-% Octenidin,
b2) 0,03 Gew.-% bis 10 Gew.-% eines Zusatzstoffs, der ausgewählt ist aus einem Aminoxid der folgenden allgemeinen Formel
R¹R²R³N-O,
wobei R¹ Methyl, Ethyl oder 2-Hydroxyethyl ist, R² Methyl, Ethyl oder 2-Hydroxyethyl ist, R¹ und R² zusammen Morpholin sein können, R³ Alkyl mit 8 bis 18 Kohlenstoffatomen ist, oder R⁴CONH(CH₂)ₙ, wobei R⁴ Alkyl mit 8 bis 18 Kohlenstoffatomen ist und n im Bereich von 1 bis 10 liegt, einer C₈- bis C₁₆-Alkylpolyglucose eines Fettalkohols oder Cocamidopropylbetain; und
b3) Wasser.

6. Kit Struktur nach Anspruch 5, **dadurch gekennzeichnet, dass** die C₈-C₁₆-Alkylpolyglucose eine Laurylpolyglucose, eine Decylpolyglucose oder eine Mischung davon ist.

7. Kitnach Anspruch 5, **dadurch gekennzeichnet, dass** das Aminoxid Cocamidopropylaminoxid ist.

8. Kit nach einem der Ansprüche 5 bis 7, **dadurch gekennzeichnet, dass** die antimikrobielle Wirkstofflösung ferner Folgendes umfasst
b4) Glycerin in einer Menge von 0,5 Gew.-% bis 10 Gew.-% und 1-(2-Ethylhexyl)glycerinether in einer Menge von 0,01 Gew.-% bis 5 Gew.-%.

## Revendications

1. Structure de type feuille textile imprégnée d'une solution d'ingrédient actif antimicrobien qui comprend
a) un matériau de support synthétique constitué de polypropylène, de polyéthylène ou d'un mélange des deux polymères, et
b) une solution d'ingrédient actif antimicrobien qui comprend :
b1) 0,02 % à 5 % en poids d'octénidine,
b2) 0,03 % à 10 % en poids d'un additif choisi parmi un oxyde d'amine de formule générale
R¹R²R³N-O,
dans laquelle R¹ est méthyle, éthyle ou 2-hydroxyéthyle, R² est méthyle, éthyle ou 2-hydroxyéthyle, R¹ et R² peuvent ensemble être la morpholine, R³ est un alkyle comportant 8 à 18 atomes de carbone ou R⁴CONH(CH₂)ₙ, où R⁴ est un alkyle comportant 8 à 18 atomes de carbone et n est dans la plage de 1 à 10, un alkyle en C₈ à C₁₆ polyglucose d'un alcool gras ou la cocamidopropylbétaine ; et
b3) de l'eau.

2. Structure de type feuille selon la revendication 1, **caractérisée en ce que** l'alkyle en C₈ à C₁₆ polyglucose est un laurylpolyglucose, un décylpolyglucose ou l'un de leurs mélanges.

3. Structure de type feuille selon la revendication 1, **caractérisée en ce que** l'oxyde d'amine est l'oxyde de cocamidopropylamine.

4. Structure de type feuille selon l'une des revendications 1 à 3, **caractérisée en ce que** la solution d'ingrédient actif antimicrobien comprend en outre b4) du glycérol en une quantité de 0,5 % à 10 % en poids et du 1-(2-éthylhexyl)glycérol éther en une quantité de 0,01 % à 5 % en poids.

5. Kit de production de structure de type feuille textile imprégnée d'une solution d'ingrédient actif antimicrobien qui comprend
a) un matériau de support synthétique constitué de polypropylène, de polyéthylène ou d'un mélange des deux polymères, et
b) une solution d'ingrédient actif antimicrobien qui comprend :
b1) 0,02 % à 5 % en poids d'octénidine,
b2) 0,03 % à 10 % en poids d'un additif choisi parmi un oxyde d'amine de formule générale
R¹R²R³N-O,
dans laquelle R¹ est méthyle, éthyle ou 2-hydroxyéthyle, R² est méthyle, éthyle ou 2-hydroxyéthyle, R¹ et R² peuvent ensemble être la morpholine, R³ est un alkyle comportant 8 à 18 atomes de carbone ou R⁴CONH(CH₂)ₙ, où R⁴ est un alkyle comportant 8 à 18 atomes de carbone et n est dans la plage de 1 à 10, un alkyle en C₈- à C₁₆-polyglucose d'un alcool gras ou la cocamidopropylbétaine ; et
b3) de l'eau.

6. Kit selon la revendication 5, **caractérisé en ce que** l'alkyle en C₈ à C₁₆ polyglucose est un laurylpolyglucose, un décylpolyglucose ou l'un de leurs mélanges.

7. Kit selon la revendication 5, **caractérisé en ce que** l'oxyde d'amine est l'oxyde de cocamidopropylamine.

8. Kit selon l'une des revendications 5 à 7, **caractérisé en ce que** la solution d'ingrédient actif antimicrobien comprend en outre
b4) du glycérol en une quantité de 0,5 % à 10 % en poids et du 1-(2-éthylhexyl)glycérol éther en une quantité de 0,01 % à 5 % en poids.
